# FASCICULE DE BREVET EUROPEEN

(11) **EP 0 528 734 B1**
(45) Date de publication et mention de la délivrance du brevet: **23.10.1996**
(21) Numéro de dépôt: 92402308.8
(22) Date de dépôt: 20.08.1992
(51) Int. Cl.: C07D 277/34, C07D 417/14, C07D 417/12, A61K 31/425, A61K 31/47

(54) **Dérivés de 2,4-thiazolidinedione, leur procédé de préparation et les compositions pharmaceutiques qui les contiennent**
Thiazolidin-2,4-Dionderivate, Verfahren zu deren Herstellung und sie enthaltende pharmazeutische Zusammensetzungen
Thiazolidin-2,4-dione derivatives, process for their preparation and pharmaceutical compositions containing them

(30) Priorité: 20.08.1991 FR 9110430
(43) Date de publication de la demande: 24.02.1993
(73) Titulaire: ADIR ET COMPAGNIE, F-92415 Courbevoie Cédex (FR)
(72) Inventeur: Nanteuil de, Guillaume, F-92150 Suresnes (FR); Duhault, Jacques, F-78290 Croissy sur Seine (FR); Ravel, Denis, F-91430 Igny (FR); Herve, Yolande, F-92800 Puteaux (FR)

(56) Documents cités:
- EP-A- 0 008 203
- EP-A- 0 193 256
- EP-A- 0 257 781
- CHEMICAL AND PHARMACEUTICAL BULLETIN, vol. 30, no. 10, Octobre 1982, TOKYO ,JP; pages 3580-3600, TAKASHI SOHDA et al.: "Studies on antidiabetic agents. II. Synthesis of 5-[4-(1-methylcyclohexylmethoxy)benzyl]thiazolidine-2,4-dione(ADD-3878) and its derivatives."
- Arzneimittel-Forschung/Drug Research (1995), Vol.45(II), Nr.11, 1176-1181

## Description

La présente invention concerne de nouveaux dérivés de 2,4-thiazolidinedione, leur procédé de préparation et les compositions pharmaceutiques qui les contiennent.

De nombreux dérivés de 2,4-thiazolidinedione ont été décrits dans l'Art Antérieur comme agents antidiabétiques. C'est le cas en particulier des composés décrits dans les brevets EP 008203, WO 8607056, EP 207581 WO 8504171 ou EP 193 256, ainsi que dans Chem. Pharm, Bull., 30, 3580, 1982.

Or les composés de la présente invention, outre le fait qu'ils soient nouveaux, se différentient des autres dérivés de 2,4-thiazolidinedione par l'intensité de leurs propriétés pharmacologiques.

En effet, l'insulinorésistance et le défaut de sécrétion de l'insuline sont les responsables de l'intolérance au glucose observée chez les patients ayant un diabète non insulino-dépendant.

Les thérapeutiques actuellement disponibles permettent de corriger essentiellement le défaut de sécrétion d'insuline sans nécessairement améliorer la sensibilité à l'insuline des tissus périphériques (muscles, tissu adipeux).

Les composés appartenant à la structure 2,4-thiazolidinedione sont capables de provoquer une baisse de la glycémie et d'améliorer la tolérance au glucose dans des modèles de diabète non insulino-dépendant sans provoquer une augmentation de sécretion d'insuline. Nos composés présentent l'avantage d'être particulièrement puissants plus spécialement par rapport à la ciglitazone, composé de référence appartenant à cette structure chimique et dont l'efficacité reste faible.

Ainsi les composés de l'invention sont utilisables dans le traitement des états diabétiques non insulinopéniques, permettant d'obtenir un meilleur contrôle de la glycémie alors que le taux d'insuline circulante diminue. La prévention de cette hyperinsulinémie relative, associée à une diminution des triglycérides circulant sous l'effet de ces produits, peut concourir à une réduction des risques de macroangiopathie.

Ces mêmes composés trouvent de plus une utilisation dans le traitement de l'hypertension chez les sujets âgés présentant une insulinorésistance associée ou non à d'autres anomalies métaboliques.

Plus spécifiquement, la présente invention concerne les dérivés de formule (I) : 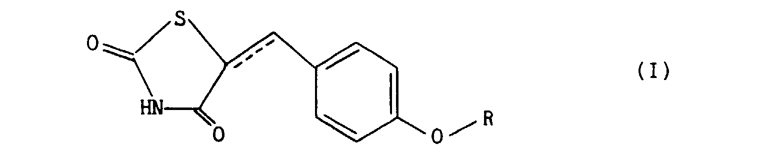 dans laquelle :
- le trait en pointillé signifie la présence ou non d'une double liaison,
- R représente l'un quelconque des groupements suivants : 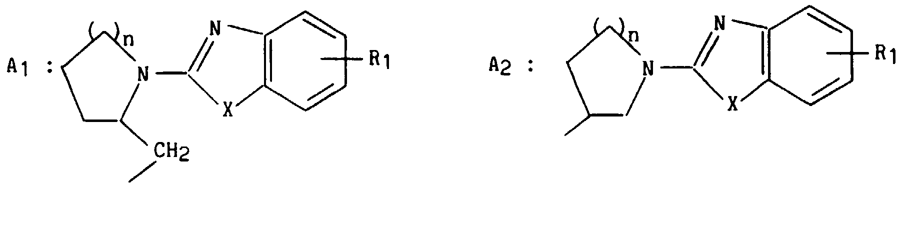 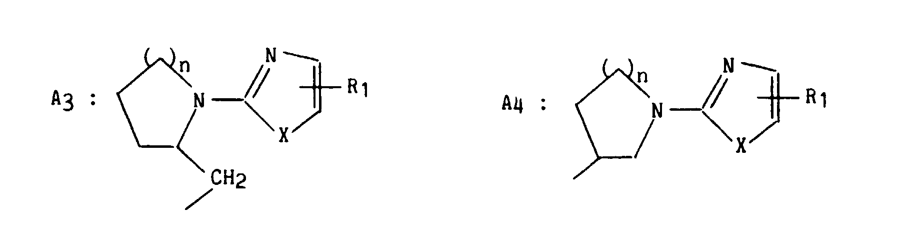
dans lesquels :
- X: représente un atome de soufre, d'oxygène ou un radical NH substitué ou non par un groupement alkyle (C₁-C₆) linéaire ou ramifié,
- R₁: représente un atome d'hydrogène, d'halogène, un groupement alkyl (C₁-C₆) linéaire ou ramifié, alkoxy (C₁-C₆) linéaire ou ramifié, trifluorométhyl ou cyano,
- n: est égal à 1, 2 ou 3,
leurs énantiomères, diastéréoisomères et épimères ainsi que leurs sels d'addition à un acide pharmaceutiquement acceptable.

Parmi les acides pharmaceutiquement acceptables, on peut citer, à titre non limitatif, les acides chlorhydrique, sulfurique, tartrique, maléique, fumarique, méthane sulfonique, camphorique, etc...

L'invention s'étend également au procédé de préparation des composés de formule (I) caractérisé :
en ce que l'on fait réagir un alcool de formule (II), sous forme racémique ou énantiomérique,

R-OH (II)

dans laquelle R représente l'un quelconque des groupements A₁ à A₄,
avec le parahydroxybenzaldéhyde de formule (III), selon une réaction décrite par Mitsunobu (Synthesis, 1981, 1) : 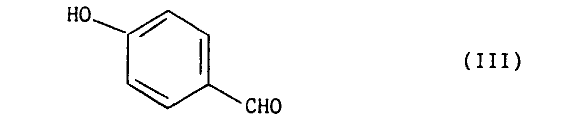 pour conduire à un dérivé de formule (IV) : 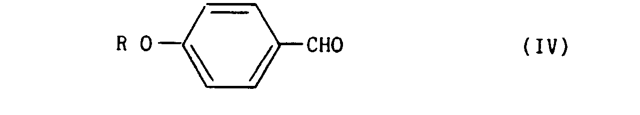 dans laquelle R a la même signification que précédemment,
que l'on soumet à l'action de la 2,4-thiazolidinedione de formule (V), en présence d'une base telle que la pipéridine : 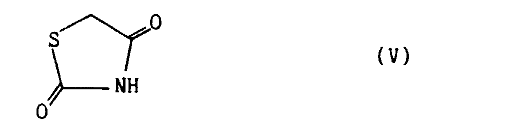 pour conduire à un composé de formule (I/a), cas particulier des composés de formule (I), 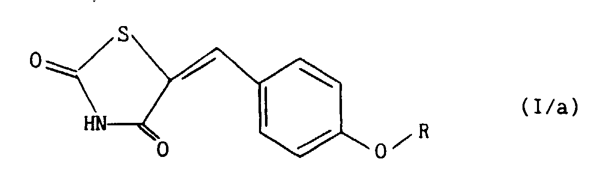 dans lequel R a la même signification que précédemment,
que l'on soumet, si on le désire, à une hydrogénation catalytique en présence de palladium sur charbon à 10 % pour obtenir un composé de formule (I/b), cas particulier des composés de formule (I), 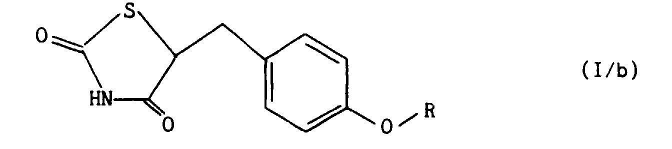 dans laquelle R a la même signification que précédemment, dérivés de formule (I/a) ou (I/b) que l'on purifie le cas échéant par une technique classique de purification, dont on sépare, si on le souhaite, les isomères, selon une technique classique de séparation et que l'on transforme, si nécessaire, en leurs sels d'addition à un acide pharmaceutiquement acceptable.

Les composés de formule (I) possèdent des propriétés pharmacologiques très intéressantes. Les dérivés de la thiazolidinedione sont généralement inactifs in vitro et chez les animaux normoglycémiques ne présentant pas d'anomalie de la tolérance au glucose. Le test pharmacologique des composés de l'invention est donc réalisé dans un modèle de diabète non insulinodépendant (NIDDM) : la souris ob/ob et dans un modèle de tolérance au glucose diminuée, associée à une hyperinsulinémie et une hyperlipémie : le rat Zucker FaFa. Les résultats obtenus lors de ces tests montrent que les composés de l'invention permettent un contrôle de la glycémie alors que les taux d'insuline circulante et de triglycérides diminuent.

En plus de ces propriétés pharmacologiques relatives à l'hyperinsulinémie, les composés de formule (I), bien que dépourvus d'activité hypotensive intrinsèque, diminuent la pression artérielle des sujets insulino-résistants et de ce fait peuvent être utilisés en thérapeutique dans le traitement de l'hypertension associée aux états d'insulino-résistance et d'autres maladies métaboliques telles que par exemple obésité, dyslipémie, hyperinsulinémie, etc... qui constituent des facteurs de risques cardiovasculaires importants (coronaropathies, macroangiopathie, etc...).

La présente invention a également pour objet les compositions pharmaceutiques renfermant comme principe actif au moins un composé de formule générale (I) ou un de ses sels d'addition à un acide pharmaceutiquement acceptable, seul ou en combinaison avec un ou plusieurs excipients ou véhicules inertes, non toxiques.

Parmi les compositions pharmaceutiques selon l'invention on pourra citer plus particulièrement celles qui conviennent pour l'administration orale, parentérale, nasale, les comprimés simples ou dragéifiés, les comprimés sublinguaux, les sachets, les paquets, les gélules, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 50 et 1000 mg pour un traitement en 1 à 3 prises par 24 heures.

Les exemples suivants illustrent l'invention et ne la limitent en aucune façon.

### EXEMPLE 1 : (-)-5-{4-[[1-(Benzothiazol-2-yl)-(2S)-pyrrolidin-2-yl]méthoxy] benzylidène}thiazolidin-2,4-dione

### STADE A : (S)-1-(Benzothiazol-2-yl)-2-hydroxyméthylpyrrolidine

15 g de (S)-(+)-2-pyrrolidineméthanol sont placés, à température ambiante, dans 100 ml d'acétonitrile anhydre en présence de 20,5 g de carbonate de potassium et de 25 g de 2-chlorobenzothiazole.

Ce mélange est porté à reflux 15 heures, puis filtré et la solution évaporée sous vide.

Le résidu est repris par 200 ml d'acide chlorhydrique 2N et la solution obtenue est lavée à l'éther.

Le solide qui précipite de la phase aqueuse acide, est filtré, lavé à l'éther et séché. Il est alors dissous dans de l'eau et la solution est amenée à pH 12 au moyen de soude concentrée.

Le produit attendu est obtenu après extraction au chlorure de méthylène, séchage et évaporation du solvant.
Point de fusion : 110°C

### STADE B : (S)-1-(Benzothiazol-2-yl)-2(4-formylphénoxyméthyl)pyrrolidine

A 190 g de triphénylphosphine dissoute dans 1,5 litre de tétrahydrofurane (THF), on ajoute goutte à goutte, à 15°C, 119 g d'azodicarboxylate d'éthyle.

Après 5 minutes à température ambiante, on ajoute, goutte à goutte rapidement, à température ambiante, 44 g de parahydroxybenzaldéhyde dans 1,5 litres de THF puis 85 g du produit décrit au stade A préalablement dissous dans 1,5 litres de THF.

Après une nuit à température ambiante, le solvant est évaporé. En triturant le résidu à l'éther, une partie de l'oxyde de triphénylphosphine formé cristallise. Le filtrat est concentré et purifié par chromatographie sur gel de silice en utilisant comme éluant un mélange éther/cyclohexane : 80/20.

Le composé solide obtenu est filtré et lavé abondamment à l'éther. Le filtrat est séché et évaporé et conduit au produit attendu sous forme d'huile.

### STADE C : (-)-5-{4-[[1-(Benzothiazol-2-yl)-(2S)-pyrrolidin-2-yl]méthoxy] benzylidène}thiazolidin-2,4-dione

30 g du composé obtenu au stade B sont placés à température ambiante dans 650 ml d'éthanol en présence de 10,3 g de thiazolidinedione et de 6 ml de pipéridine. Le mélange est porté à reflux 24 heures. Le produit attendu précipite au refroidissement, est filtré et lavé à l'isopropanol et à l'éther.
Point de fusion : 192-194°C

| Microanalyse élémentaire : | | | | |
|---|---|---|---|---|
| | C % | H % | N % | S % |
| calculé | 60,39 | 4,38 | 9,60 | 14,66 |
| trouvé | 60,38 | 4,33 | 9,62 | 13,66 |

Pouvoir rotatoire : [α]²⁰ _{D} = - 141,7° (c = 10 mg/ml, DMSO)

### EXEMPLE 2 : (+)-5-{4-[[1-(Benzothiazol-2-yl)-(2R)-pyrrolidin-2-yl]méthoxy] benzylidène}thiazolidin-2,4-dione

### STADE A : (R)-1-(Benzothiazol-2-yl)-2-hydroxyméthylpyrrolidine

Le produit attendu est obtenu en procédant comme au stade A de l'exemple 1, mais en remplacant le (S)-(+)-2-pyrrolidine méthanol par le R-(-)-2-pyrrolidine méthanol.
Point de fusion : 110°C

### STADE B : (R)-1-(Benzothiazol-2-yl)-2-(4-formylphénoxyméthyl)pyrrolidine

Ce stade est identique au stade B de l'exemple 1.

### STADE C : (+)-5-{4-[[1-(Benzothiazol-2-yl)-(2R)-pyrrolidin-2-yl]méthoxy] benzylidène}thiazolidin-2,4-dione

Ce stade est identique au stade C de l'exemple 1.
Point de fusion : 214-215°C

| Microanalyse élémentaire : | | | | |
|---|---|---|---|---|
| | C % | H % | N % | S % |
| calculé | 60,39 | 4,38 | 9,60 | 14,66 |
| trouvé | 60,02 | 4,34 | 9,76 | 14,57 |

Pouvoir rotatoire : [α]²⁰ _{D} = + 135,9° (c = 10 mg/ml, DMSO)

### EXEMPLE 3 : (-)-5-{4-[[1-(Benzoxazol-2-yl)-(2S)-pyrrolidin-2-yl]méthoxy] benzylidène}thiazolidin-2,4-dione

### STADE A : (S)-1-(Benzoxazol-2-yl)-2-hydroxyméthylpyrrolidine

Le produit attendu est obtenu en procédant comme au stade A de l'exemple 1, mais en remplacant le 2-chlorobenzothiazole par le 2-chlorobenzoxazole.
Point de fusion : 88°C

### STADE B : (S)-1-(Benzoxazol-2-yl)-2-(4-formylphénoxyméthyl)pyrrolidine

Ce stade est identique au stade B de l'exemple 1.

### STADE C : (-)-5-{4-[[1-(Benzoxazol-2-yl)-(2S)-pyrrolidin-2-yl]méthoxy] benzylidène}thiazolidin-2,4-dione

Ce stade est identique au stade C de l'exemple 1.
Point de fusion : 214°C

| Microanalyse élémentaire : | | | | |
|---|---|---|---|---|
| | C % | H % | N % | S % |
| calculé | 62,69 | 4,69 | 10,21 | 7,61 |
| trouvé | 62,69 | 4,75 | 10,21 | 7,47 |

Pouvoir rotatoire : [α]²⁰ _{D} = - 156,2° (c = 10 mg/ml, DMSO)

### EXEMPLE 11 : (-)-5-{4-[[1-(6-Chlorobenzothiazol-2-yl)-(2S)-pyrrolidin-2-yl]méthoxy]benzylidène}thiazolidin-2,4-dione

### STADE A : (S)-1-(6-Chlorobenzothiazol-2-yl)-2-hydroxyméthylpyrrolidine

### STADE B : (S)-1-(6-Chlorobenzothiazol-2-yl)-2-(4-formylphénoxyméthyl) pyrrolidine

### STADE C : (-)-5-{4-[[1-(6-Chlorobenzothiazol-2-yl)-(2S)-pyrrolidin-2-yl]méthoxy]benzylidène}thiazolidin-2,4-dione

### EXEMPLE 12 : (-)-5-{4-[[1-(Thiazol-2-yl)-(2S)-pyrrolidin-2-yl]méthoxy] benzylidène}thiazolidin-2,4-dione

### STADE A : (S)-1-(Thiazol-2-yl)-2-hydroxyméthylpyrrolidine

### STADE B : (S)-1-(Thiazol-2-yl)-2-(4-formylphénoxyméthyl)pyrrolidine

### STADE C : (-)-5-{4-[[1-(Thiazol-2-yl)-(2S)-pyrrolidin-2-yl]méthoxy] benzylidène}thiazolidin-2,4-dione

### EXEMPLE 13 : 5-{4-[[1-(Benzothiazol-2-yl)pyrrolidin-3-yl]oxy]benzylidène} thiazolidin-2,4-dione

### STADE A : 1-(Benzothiazol-2-yl)-3-hydroxypyrrolidine

### STADE B : 1-(Benzothiazol-2-yl)-3-(4-formylphénoxy)pyrrolidine

### STADE C : 5-{4-[[1-(Benzothiazol-2-yl)pyrrolidin-3-yl]oxy]benzylidène} thiazolidin-2,4-dione

### EXEMPLE 15 : (-)-5-{4-[[1-(Benzothiazol-2-yl)-(2S)-pyrrolidin-2-yl] méthoxy]benzyl}thiazolidin-2,4-dione

Le composé décrit dans l'exemple 1 est hydrogéné à 80-100° sous 60-80 kg pendant 24 heures dans le dioxane, en présence de charbon palladié à 10 %.

La réaction est poursuivie jusqu'à disparition du produit de départ.

Le produit attendu est obtenu après filtration du catalyseur.
Point de fusion : 95°C

| Microanalyse élémentaire : | | | | |
|---|---|---|---|---|
| | C % | H % | N % | S % |
| calculé | 60,12 | 4,82 | 9,56 | 14,59 |
| trouvé | 59,34 | 5,15 | 9,43 | 14,17 |

Pouvoir rotatoire : [α]²⁰ _{D} = - 99,0° (c = 10 mg/ml, DMSO)

### EXEMPLE 16 : (+)-5-{4-[[1-(Benzothiazol-2-yl)-(2R)-pyrrolidin-2-yl] méthoxy]benzyl}thiazolidin-2,4-dione

Le produit attendu est obtenu en procédant comme dans l'exemple 15 mais en hydrogénant le composé décrit dans l'exemple 2.
Point de fusion : 94,6°C

| Microanalyse élémentaire : | | | | |
|---|---|---|---|---|
| | C % | H % | N % | S % |
| calculé | 60,12 | 4,82 | 9,56 | 14,59 |
| trouvé | 60,79 | 4,91 | 9,56 | 13,94 |

Pouvoir rotatoire : [α]²⁰ _{D} = + 105,4° (c = 10 mg/ml, DMSO)

### EXEMPLE 17 : (-)-5-{4-[[1-(Benzoxazol-2-yl)-(2S)-pyrrolidin-2-yl]méthoxy] benzyl}thiazolidin-2,4-dione

Le produit attendu est obtenu en procédant comme dans l'exemple 15 mais en hydrogénant le composé décrit dans l'exemple 3.
Point de fusion : 95-107°C

| Microanalyse élémentaire : | | | | |
|---|---|---|---|---|
| | C % | H % | N % | S % |
| calculé | 62,40 | 5,00 | 9,92 | 7,57 |
| trouvé | 62,44 | 5,31 | 10,04 | 7,36 |

Pouvoir rotatoire : [α]²⁰ _{D} = - 116,7° (c = 10 mg/ml, DMSO)

### EXEMPLE 25 : (-)-5-{4-[[1-(6-Chlorobenzothiazol-2-yl)-(2S)-pyrrolidin-2-yl]méthoxy]benzyl}thiazolidin-2,4-dione

Le produit attendu est obtenu en procédant comme dans l'exemple 15 mais en hydrogénant le composé décrit dans l'exemple 11.

### EXEMPLE 26 : (-)-5-{4-[[1-(Thiazol-2-yl)-(2S)-pyrrolidin-2-yl]méthoxy] benzyl}thiazolidin-2,4-dione

Le produit attendu est obtenu en procédant comme dans l'exemple 15 mais en hydrogénant le composé décrit dans l'exemple 12.

### EXEMPLE 27 : 5-{4-[[1-(Benzothiazol-2-yl)pyrrolidin-3-yl]oxy]benzyl} thiazolidin-2,4-dione

Le produit attendu est obtenu en procédant comme dans l'exemple 15 mais en hydrogénant le composé décrit dans l'exemple 13.

### Etude pharmacologique des composés de l'invention

### EXEMPLE 38 : Etude de l'activité des composés de l'invention sur un modèle de diabète non insuline-dépendant (NIDDM)

Les animaux (souris ob/ob) sont traités chaque jour, pendant 4 jours, en administrant par voie orale les composés de l'invention en suspension dans une solution à 20 % de gomme du Sénégal.

Le 5ème Jour, le sang est prélevé par ponction du sinus orbital et la glycémie est déterminée. Les composés de référence utilisés sont la ciglitazone et le composé CS045 (San Kyo).

Dans ces conditions, les composés de l'invention ont un pouvoir hypoglycémiant 5 à 20 fois supérieur à celui de la ciglitazone et de 2 à 10 fois supérieur au composé CS045.

Le tableau ci-dessous rassemble les résultats des doses à administrer qui provoquent un même effet hypoglycémiant pour les composés de l'invention et les composés de référence :

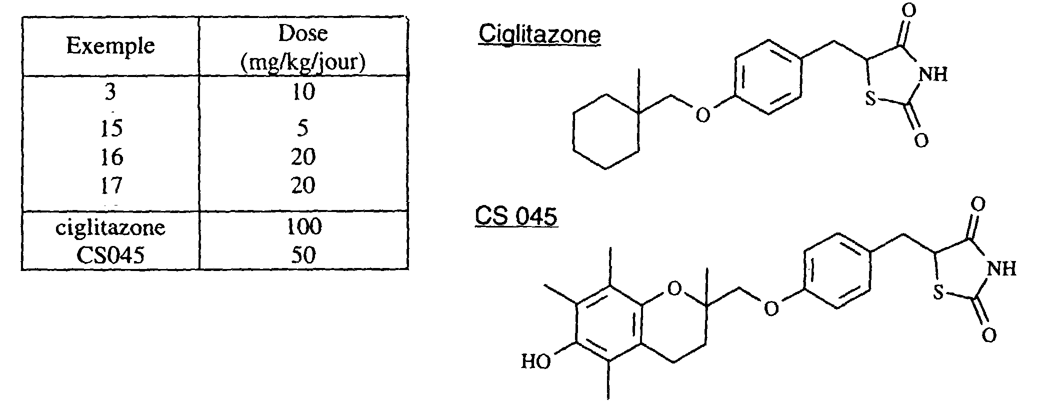

### EXEMPLE 39 : Etude de l'activité des composés de l'invention sur un modèle de tolérance au glucose diminuée associée à une hyperinsulinémie et une hyperlipémie

Les animaux (rats Zucker Fa/Fa mâles) sont traités, chaque jour, pendant 10 jours en administrant, par voie orale, les composés de l'invention à la dose de 5 mg/kg/jour en suspension dans une solution à 20 % de gomme du Sénégal. Le 11ème jour, les animaux sont sacrifiés et le sang recueilli afin de déterminer la glycémie, les triglycérides plasmatiques et l'insuline immuno-réactive. D'autre part, les animaux sont pesés avant et après traitement.

Dans ces conditions, les composés de l'invention n'ont pas d'influence sur le taux de glucose circulant mais diminuent le taux de triglycérides plasmatiques ainsi que celui de l'insuline immuno-réactive. Cette activité est égale ou supérieure à celle d'autres dérivés de thiazolidinedione de référence.

### Composition pharmaceutique

### EXEMPLE 40 :

| **Comprimé : formule de préparation pour 1000 comprimés dosés à 50 mg** | |
|---|---|
| (-)-5-{4-[[1-(Benzothiazol-2-yl)-(2S)-pyrrolidin-2-yl]méthoxy]benzyl}thiazolidin-2,4-dione | 50 g |
| Hydroxy propyl cellulose | 2 g |
| Amidon de blé | 10 g |
| Lactose | 100 g |
| Stéarate de magnésium | 3 g |
| Talc | 3 g |

## Revendications (Revendications pour l'(les) Etat(s) contractant(s) suivant(s): AT, BE, CH, DE, DK, FR, GB, IE, IT, LI, LU, NL, PT, SE)

1. Composés de formule (I), 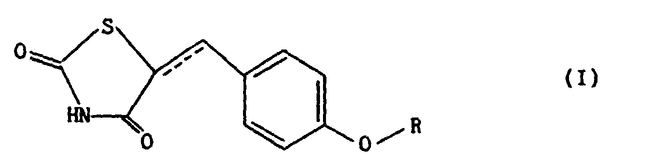 dans laquelle :
- le trait en pointillé signifie la présence ou non d'une double liaison,
- R représente l'un quelconque des groupements suivants : 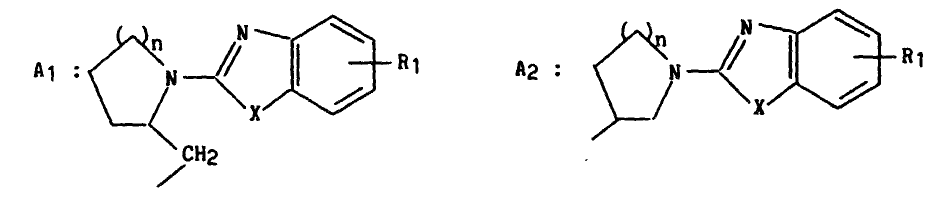 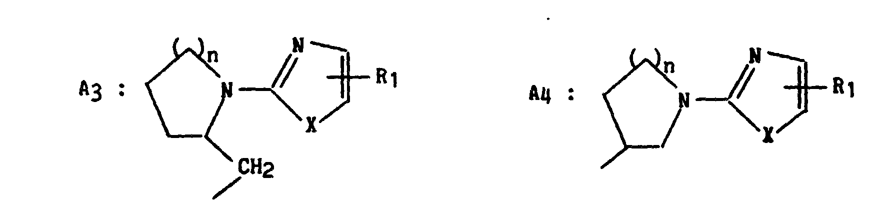
dans lesquels :
X représente un atome de soufre, d'oxygène ou un radical NH substitué ou non par un groupement alkyle (C₁-C₆) linéaire ou ramifié,
R₁ représente un atome d'hydrogène, d'halogène, un groupement alkyl (C₁-C₆) linéaire ou ramifié, alkoxy (C₁-C₆) linéaire ou ramifié, trifluorométhyl ou cyano,
n est égal à 1, 2 ou 3,
leurs énantiomères, diastéréoisomères et épimères ainsi que leurs sels d'addition à un acide pharmaceutiquement acceptable.

2. Composés de formule (I) selon la revendication 1 tels que R représente un groupement [1-(benzothiazol-2-yl )pyrrolidin-2-yl)méthyle, substitué ou non, ses énantiomères, diastéréoisomères et épimères ainsi que ses sels d'addition à un acide pharmaceutiquement acceptable.

3. Composés de formule (I) selon la revendication 1 tels que R représente un groupement [1-(benzoxazol-2-yl)pyrrolidin-2-yL]méthyle, substitué ou non, ses énantiomères, diastéréoisomères et épimères ainsi que ses sels d'addition à un acide pharmaceutiquement acceptable.

4. Procédé de préparation des composés de formule (I) selon la revendication (1), caractérisé :
en ce que l'on fait réagir un alcool de formule (II), sous forme racémique ou énantiomérique,
R-OH (II)
dans laquelle R représente l'un quelconque des groupements A₁ à A₄
avec le parahydroxybenzaldéhyde de formule (III) : 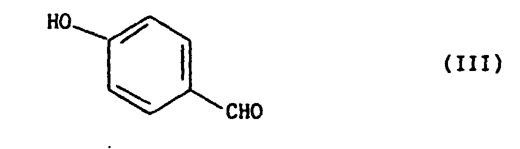 pour conduire à un dérivé de formule (IV) : 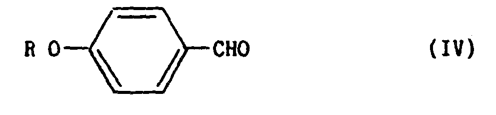 dans laquelle R a la même signification que précédemment,
que l'on soumet à l'action de la 2,4-thiazolidinedione de formule (V), en présence d'une base telle que la pipéridine : 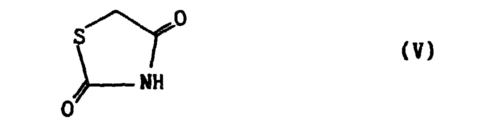 pour conduire à un composé de formule (I/a), cas particulier des composés de formule (I), 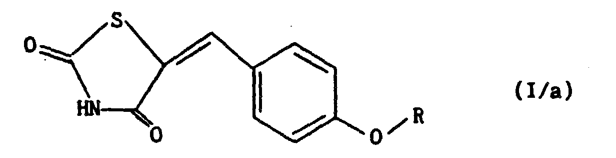 dans lequel R a la même signification que précédemment,
que l'on soumet, si on le désire, à une hydrogénation catalytique en présence de palladium sur charbon à 10 % pour obtenir un composé de formule (I/b), cas particulier des composés de formule (I), 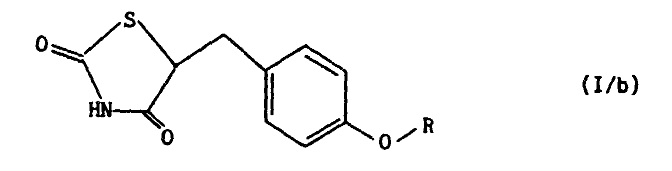 dans laquelle R a la même signification que précédemment,
dérivés de formule (I/a) ou (I/b) que l'on purifie, le cas échéant, par une technique classique de purification, dont on sépare, si on le souhaite, les isomères par une technique classique de séparation et que l'on transforme, si nécessaire, en leurs sels d'addition à un acide pharmaceutiquement acceptable.

5. Compositions pharmaceutiques contenant comme principe actif au moins un composé selon l'une quelconque des revendications 1 à 3, seul ou en combinaison avec un ou plusieurs excipients ou véhicules inertes, non toxiques, pharmaceutiquement acceptables.

6. Compositions pharmaceutiques selon la revendication 5 contenant au moins un principe actif selon l'une quelconque des revendications 1 à 3 utiles dans le traitement des diabètes non insulinopéniques associés ou non à une hypertension.

## Revendications (Revendications pour l'(les) Etat(s) contractant(s) suivant(s): ES, GR)

1. Procédé de préparation des composés de formule (I), 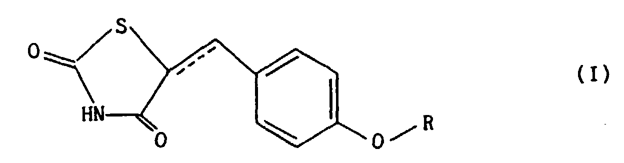 dans laquelle :
- le trait en pointillé signifie la présence ou non d'une double liaison,
- R représente l'un quelconque des groupements suivants : 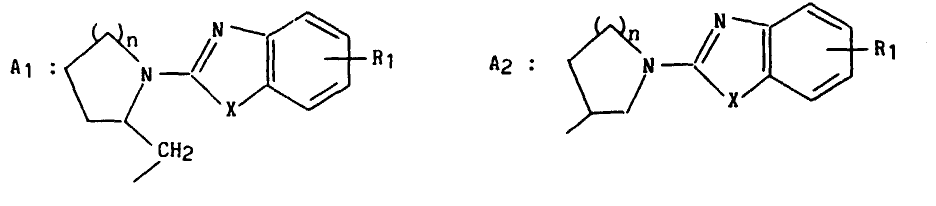 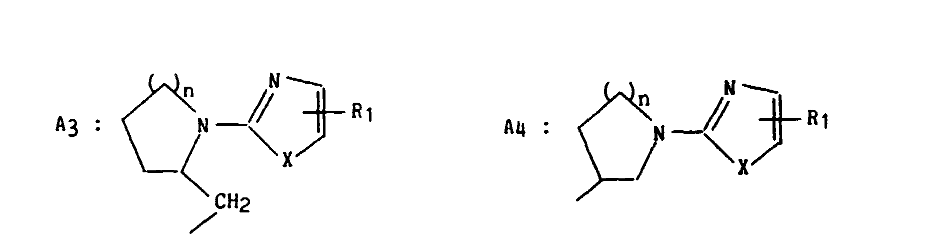
dans lesquels :
X représente un atome de soufre, d'oxygène ou un radical NH substitué ou non par un groupement alkyle (C₁-C₆) linéaire ou ramifié,
R₁ représente un atome d'hydrogène, d'halogène, un groupement alkyl (C₁-C₆) linéaire ou ramifié, alkoxy (C₁-C₆) linéaire ou ramifié, trifluorométhyl ou cyano,
n est égal à 1, 2 ou 3,
leurs énantioméres, diastéréoisomères et épimères ainsi que leurs sels d'addition à un acide pharmaceutiquement acceptable,
caractérisé :
en ce que l'on fait réagir un alcool de formule (II), sous forme racémique ou énantiomérique,
R-OH (II)
dans laquelle R représente l'un quelconque des groupements A₁ à A₄
avec le parahydroxybenzaldéhyde de formule (III) : 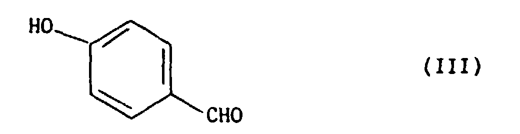 pour conduire à un dérivé de formule (IV) : 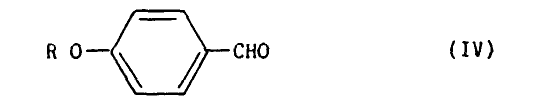 dans laquelle R a la même signification que précédemment,
que l'on soumet à l'action de la 2,4-thiazolidinedione de formule (V), en présence d'une base telle que la pipéridine : 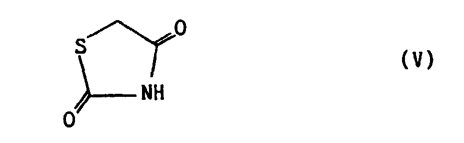 pour conduire à un composé de formule (I/a), cas particulier des composés de formule (I), 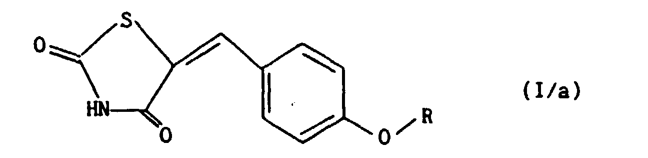 dans lequel R a la même signification que précédemment,
que l'on soumet, si on le désire, à une hydrogénation catalytique en présence de palladium sur charbon à 10 % pour obtenir un composé de formule (I/b), cas particulier des composés de formule (I), 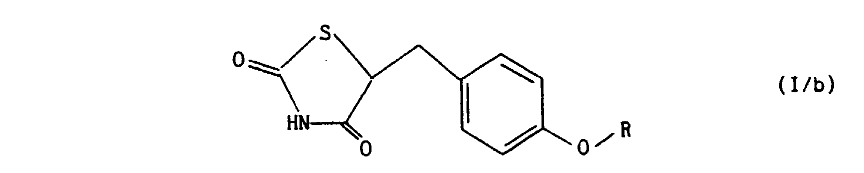 dans laquelle R a la même signification que précédemment,
dérivés de formule (I/a) ou (I/b) que l'on purifie le cas échéant par une technique classique de purification, dont on sépare, si on le souhaite, les isomères, selon une technique classique de séparation et que l'on transforme, si nécessaire, en leurs sels d'addition à un acide pharmaceutiquement acceptable.

2. Procédé de préparation des composés de formule (I) selon la revendication 1 tels que R représente un groupement [1-(benzothiazol-2-yl)pyrrolidin-2-yl]méthyle, substitué ou non, ses énantiomères, diastéréoisomères et épimères ainsi que ses sels d'addition à un acide pharmaceutiquement acceptable.

3. Procédé de préparation des composés de formule (I) selon la revendication 1 tels que R représente un groupement [1-(benzoxazol-2-yl)pyrrolidin-2-yl]méthyle, substitué ou non, ses énantiomères, diastéréoisomères et épimères ainsi que ses sels d'addition à un acide pharmaceutiquement acceptable.

## Patentansprüche (Patentansprüche für folgende(n) Vertragsstaat(en): AT, BE, CH, DE, DK, FR, GB, IE, IT, LI, LU, NL, PT, SE)

1. Verbindungen der Formel (I) 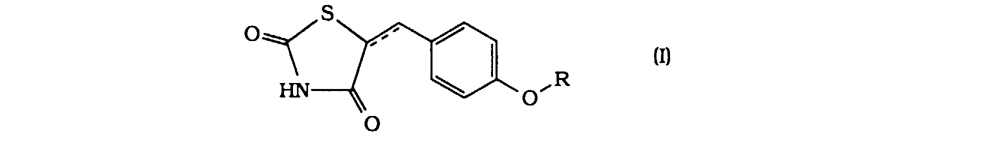 in der:
- die gestrichelte Linie für eine gegebenenfalls vorhandene Doppelbindung steht,
- R eine der folgenden Gruppe bedeutet: 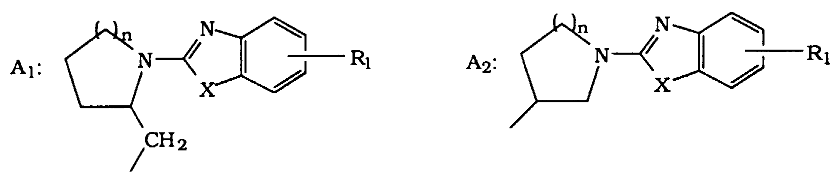 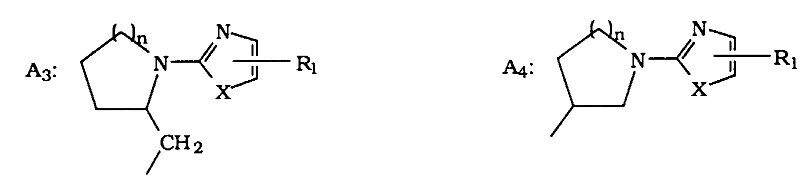
in denen:
X ein Schwefelatom. Sauerstoffatom oder eine gegebenenfalls durch eine geradkettige oder verzweigte (C₁-C₆)-Alkylgruppe substituierte NH-Gruppe.
R₁ ein Wasserstoffatom, ein Halogenatom, eine geradkettige oder verzweigte (C₁-C₆)-Alkylgruppe, eine geradkettige oder verzweigte (C₁-C₆)-Alkoxygruppe, eine Trifluormethylgruppe oder eine Cyanogruppe und
n 1, 2 oder 3 bedeuten.
deren Enantiomere. Diastereoisomere und Epimere sowie deren Additionssalze mit einer pharmazeutisch annehmbaren Säure.

2. Verbindungen der Formel (I) nach Anspruch 1, worin R eine gegebenenfalls substituierte [1-(Benzothiazol-2-yl)-pyrrolidin-2-yl]-methylgruppe bedeutet, deren Enantiomere, Diastereoisomere und Epimere sowie deren Additionssalze mit einer pharmazeutisch annehmbaren Säure.

3. Verbindungen der Formel I nach Anspruch 1, worin R eine gegebenenfalls substituierte [1-(Benzoxazol-2-yl)-pyrrolidin-2-yl]-methylgruppe bedeutet, deren Enantiomere, Diastereoisomere und Epimere sowie deren Additionssalze mit einer pharmazeutisch annehmbaren Säure.

4. Verfahren zur Herstellung der Verbindungen der Formel (I) nach Anspruch 1, **dadurch gekennzeichnet**, daß man einen Alkohol der Formel (II) in racemischer oder enantiomerer Form
R- OH (II)
in der R eine der Gruppen A₁ bis A₄ darstellt, mit p-Hydroxybenzaldehyd der Formel (III): 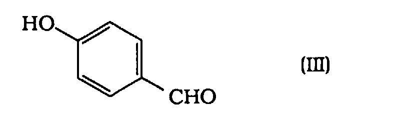 umsetzt zur Bildung eines Derivats der Formel (IV): 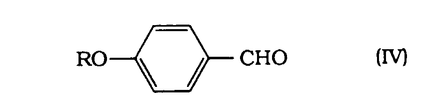 in der R die oben angegebenen Bedeutungen besitzt,
welches man der Einwirkung von 2,4-Thiazolidindion der Formel (V): 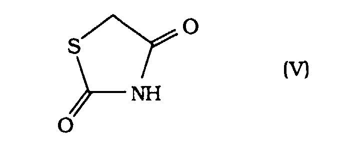 in Gegenwart einer Base, wie Piperidin, unterwirft zur Bildung einer Verbindung der Formel (I/a), einem Sonderfall der Verbindungen der Formel (I) 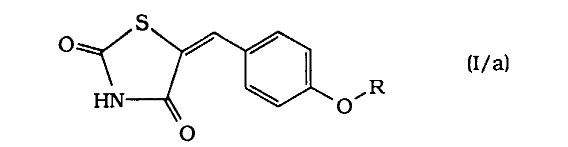 in der R die oben angegebenen Bedeutungen besitzt, welche man gewünschtenfalls einer katalytischen Hydrierung in Gegenwart von 10 % Palladium-auf-Kohlenstoff unterwirft zur Bildung einer Verbindung der Formel (I/b), einem Sonderfall der Verbindungen der Formel (I) 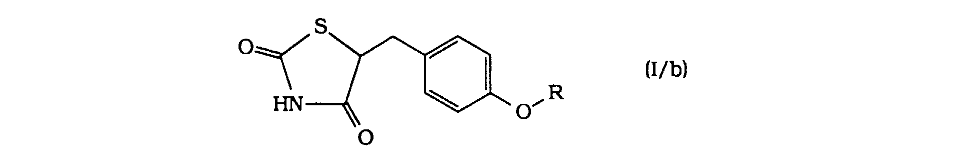 in der R die oben angegebenen Bedeutungen besitzt, welche Derivate der Formel (I/a) oder (I/b) man gegebenenfalls mit Hilfe einer klassischen Reinigungstechnik reinigt, gewünschtenfalls mit Hilfe einer klassischen Trennmethode in die Isomeren auftrennt und erforderlichenfalls mit einer pharmazeutisch annehmbaren Säure in ihre Additionssalze überführt.

5. Pharmazeutische Zubereitungen, enthaltend als Wirkstoff mindestens eine Verbindung nach einem der Ansprüche 1 bis 3 allein oder in Kombination mit einem oder mehreren inerten, nichttoxischen, pharmazeutisch annehmbaren Trägermaterialien oder Bindemitteln.

6. Pharmazeutische Zubereitungen nach Anspruch 5, enthaltend mindestens einen Wirkstoff nach einem der Ansprüche 1 bis 3 zur Behandlung des gegebenenfalls mit einer Hypertension verknüpften nicht-insulinopenen Diabetes.

## Patentansprüche (Patentansprüche für folgende(n) Vertragsstaat(en): ES, GR)

1. Verfahren zur Herstellung der Verbindungen der Formel (I) 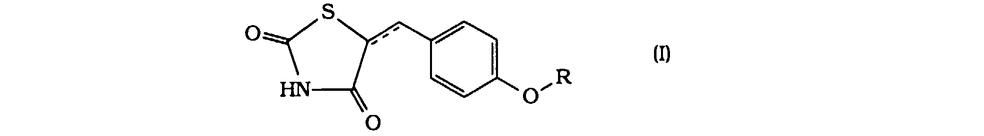 in der:
- die gestrichelte Linie für eine gegebenenfalls vorhandene Doppelbindung steht,
- R eine der folgenden Gruppe bedeutet: 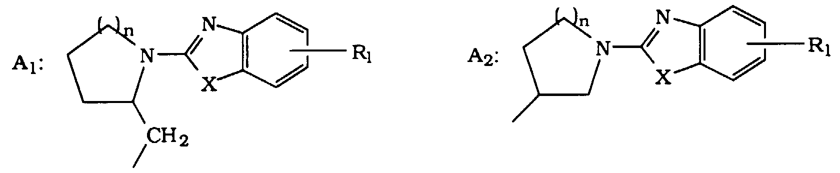 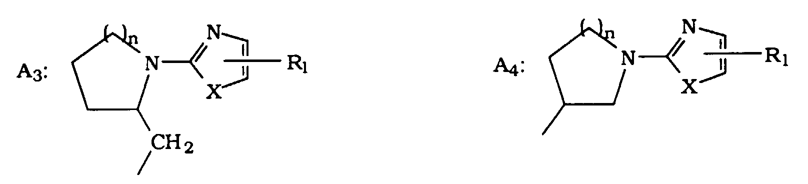
in denen:
X ein Schwefelatom, Sauerstoffatom oder eine gegebenenfalls durch eine geradkettige oder verzweigte (C₁-C₆)-Alkylgruppe substituierte NH-Gruppe,
R₁ ein Wasserstoffatom, ein Halogenatom, eine geradkettige oder verzweigte (C₁-C₆)-Alkylgruppe, eine geradkettige oder verzweigte (C₁-C₆)-Alkoxygruppe, eine Trifluormethylgruppe oder eine Cyanogruppe und
n 1, 2 oder 3 bedeuten,
von deren Enantiomeren, Diastereoisomeren und Epimeren sowie von deren pharmazeutisch annehmbaren Säureadditionssalzen, **dadurch gekennzeichnet**, daß man einen Alkohol der Formel (II) in racemischer oder enantiomerer Form
R- OH (II)
in der R eine der Gruppen A₁ bis A₄ darstellt, mit p-Hydroxybenzaldehyd der Formel (III): 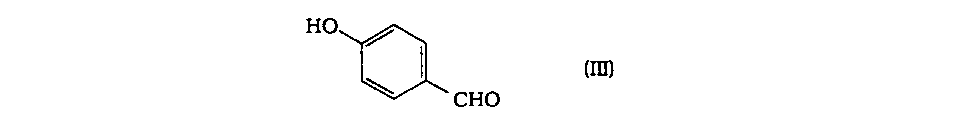 umsetzt zur Bildung eines Derivats der Formel (IV): 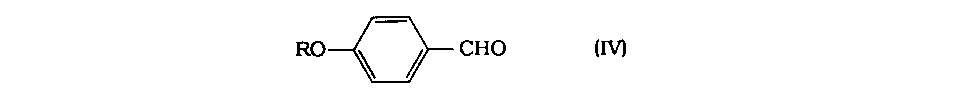 in der R die oben angegebenen Bedeutungen besitzt,
welches man der Einwirkung von 2,4-Thiazolidindion der Formel (V): 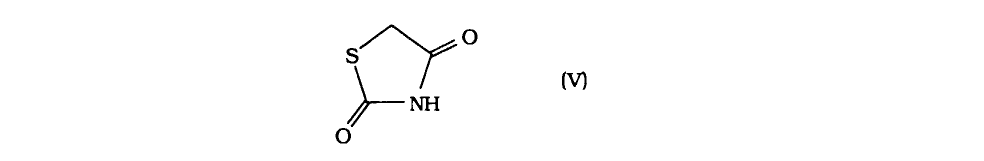 in Gegenwart einer Base, wie Piperidin, unterwirft zur Bildung einer Verbindung der Formel (I/a), einem Sonderfall der Verbindungen der Formel (I) 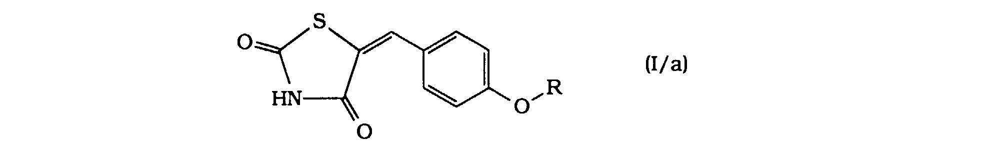 in der R die oben angegebenen Bedeutungen besitzt, welche man gewünschtenfalls einer katalytischen Hydrierung in Gegenwart von 10 % Palladium-auf-Kohlenstoff unterwirft zur Bildung einer Verbindung der Formel (I/b), einem Sonderfall der Verbindungen der Formel (I) 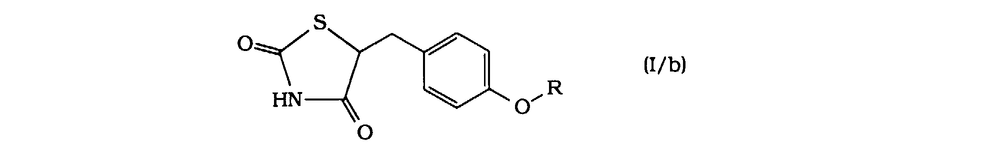 in der R die oben angegebenen Bedeutungen besitzt, welche Derivate der Formel (I/a) oder (I/b) man gegebenenfalls mit Hilfe einer klassischen Reinigungstechnik reinigt, gewünschtenfalls mit Hilfe einer klassischen Trennmethode in die Isomeren auftrennt und erforderlichenfalls mit einer pharmazeutisch annehmbaren Säure in ihre Additionssalze überführt.

2. Verfahren zur Herstellung der Verbindungen der Formel (I) nach Anspruch 1, worin R eine gegebenenfalls substituierte [1-(Benzothiazol-2-yl)-pyrrolidin-2-yl]-methylgruppe bedeutet, von deren Enantiomeren, Diastereoisomeren und Epimeren sowie von deren Additionssalzen mit einer pharmazeutisch annehmbaren Säure.

3. Verfahren zur Herstellung der Verbindungen der Formel I nach Anspruch 1, worin R eine gegebenenfalls substituierte [1-(Benzoxazol-2-yl)-pyrrolidin-2-yl]-methylgruppe bedeutet, von deren Enantiomeren, Diastereoisomeren und Epimeren sowie von deren Additionssalzen mit einer pharmazeutisch annehmbaren Säure.

## Claims (Claims for the following Contracting State(s): AT, BE, CH, DE, DK, FR, GB, IE, IT, LI, LU, NL, PT, SE)

1. Compounds of formula (I) 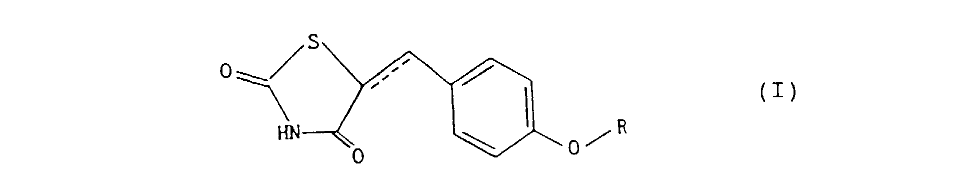 wherein:
- the broken line indicates the presence or absence of a double bond,
- R represents any one of the following groupings: 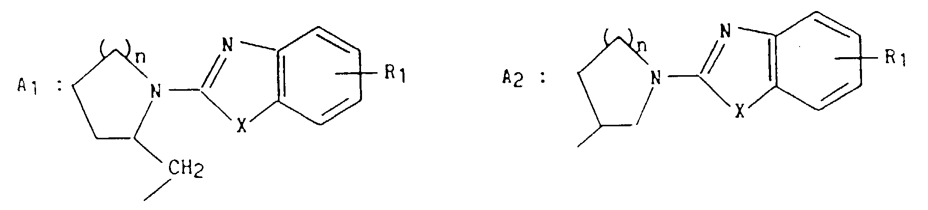 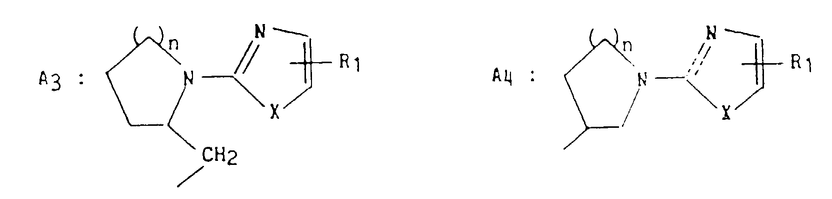
wherein:
X represents a sulphur atom, an oxygen atom or an NH radical that is unsubstituted or substituted by a linear or branched (C₁-C₆)alkyl group,
R₁ represents a hydrogen atom, a halogen atom, or a linear or branched (C₁-C₆)alkyl, linear or branched (C₁-C₆)alkoxy, trifluoromethyl or cyano group, and
n is 1, 2 or 3,
the enantiomers, diastereoisomers and epimers thereof and also the addition salts thereof with a pharmaceutically acceptable acid.

2. Compounds of formula (I) according to claim 1 wherein R represents a substituted or unsubstituted [1-(benzothiazol-2-yl)pyrrolidin-2-yl]methyl group, the enantiomers, diastereoisomers and epimers thereof and also the addition salts thereof with a pharmaceutically acceptable acid.

3. Compounds of formula (I) according to claim 1 wherein R represents a substituted or unsubstituted [1-(benzoxazol-2-yl)pyrrolidin-2-yl]methyl group, the enantiomers, diastereoisomers and epimers thereof and also the addition salts thereof with a pharmaceutically acceptable acid.

4. Process for the preparation of the compounds of formula (I) according to claim 1, characterised in that an alcohol of formula (II) in racemic or enantiomeric form:
R - OH (II),
wherein R represents any one of the groupings A₁ to A₄, is reacted with para-hydroxybenzaldehyde of formula (III): 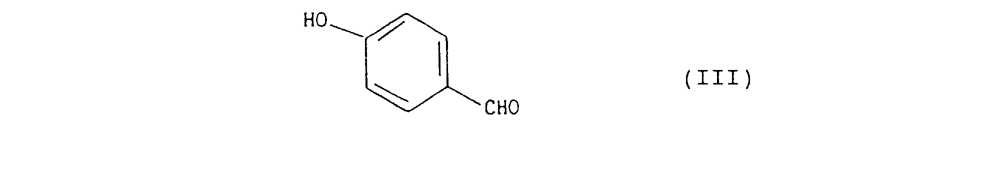 to yield a compound of formula (IV): 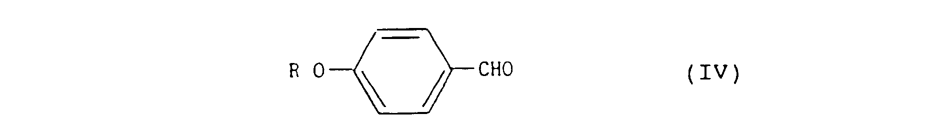 wherein R is as defined hereinbefore,
which is subjected to the action of the 2,4-thiazolidinedione of formula (V): 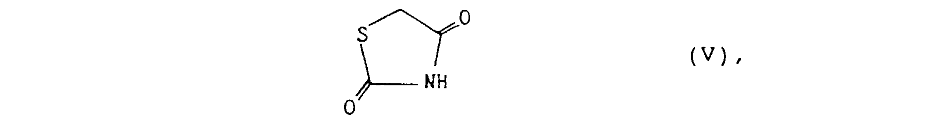 in the presence of a base, such as piperidine, to yield a compound of formula (I/a), a particular case of the compounds of formula (I): 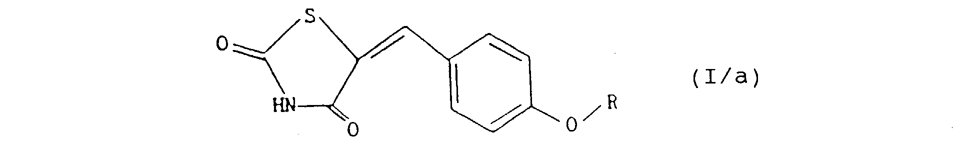 wherein R is as defined hereinbefore,
which, if desired, is subjected to catalytic hydrogenation in the presence of 10% palladium-on-carbon to obtain a compound of formula (I/b), a particular case of the compounds of formula (I): 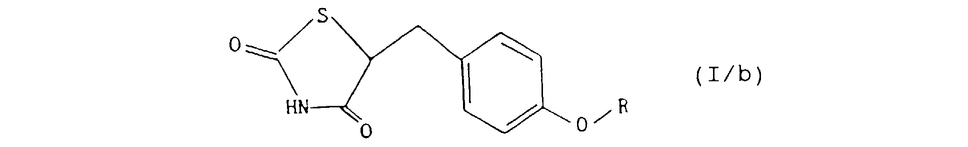 wherein R is as defined hereinbefore,
which compounds of formula (I/a) and (I/b) are purified,
where appropriate, using a conventional purification method, and are separated into their isomers, if desired, using a conventional separating method, and are converted, if necessary, into their addition salts with a pharmaceutically acceptable acid.

5. Pharmaceutical compositions comprising as active ingredient at least one compound according to any one of claims 1 to 3, on its own or in combination with one or more pharmaceutically acceptable, non-toxic, inert excipients or carriers.

6. Pharmaceutical compositions according to claim 5 comprising at least one active ingredient according to any one of claims 1 to 3 for use in the treatment of non-insulinopenic diabetes that is associated or unassociated with hypertension.

## Claims (Claims for the following Contracting State(s): ES, GR)

1. Process for the preparation of the compounds of formula (I) 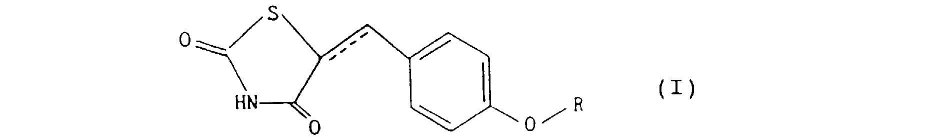 wherein:
- the broken line indicates the presence or absence of a double bond,
- R represents any one of the following groupings: 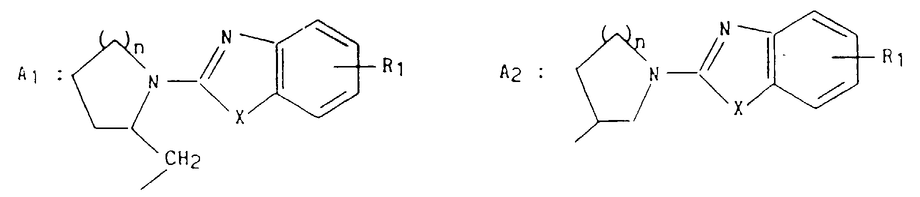 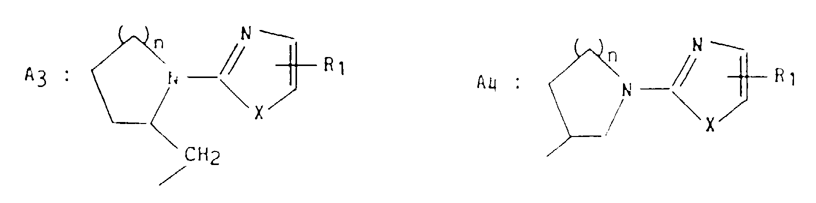
wherein:
X represents a sulphur atom, an oxygen atom or an NH radical that is unsubstituted or substituted by a linear or branched (C₁-C₆)alkyl group,
R₁ represents a hydrogen atom, a halogen atom, or a linear or branched (C₁-C₆)alkyl, linear or branched (C₁-C₆)alkoxy, trifluoromethyl or cyano group, and
n is 1, 2 or 3,
the enantiomers, diastereoisomers and epimers thereof and also the addition salts thereof with a pharmaceutically acceptable acid,
characterised in that an alcohol of formula (II) in racemic or enantiomeric form:
R - OH (II),
wherein R represents any one of the groupings A₁ to A₄, is reacted with para-hydroxybenzaldehyde of formula (III): 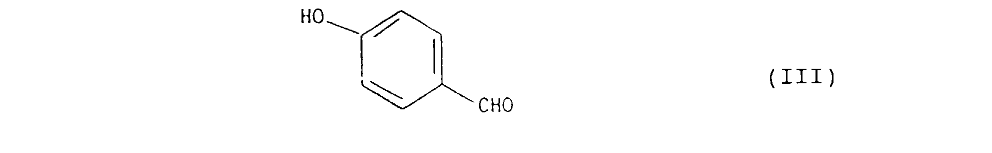 to yield a compound of formula (IV): 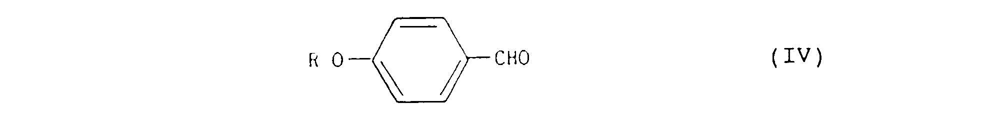 wherein R is as defined hereinbefore,
which is subjected to the action of the 2,4-thiazolidinedione of formula (V): 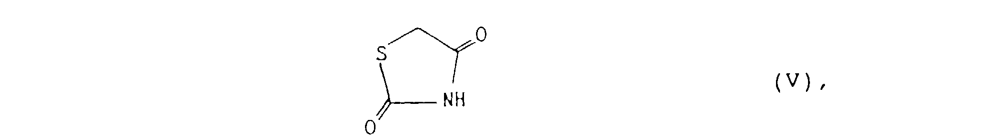 in the presence of a base, such as piperidine, to yield a compound of formula (I/a), a particular case of the compounds of formula (I), 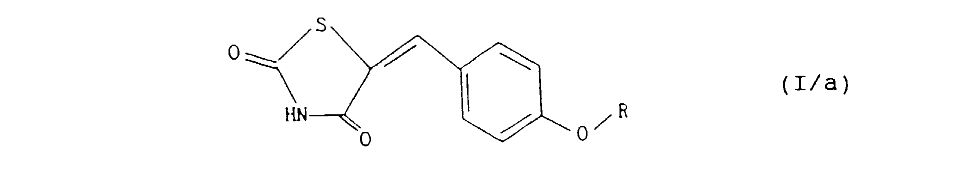 wherein R is as defined hereinbefore,
which, if desired, is subjected to catalytic hydrogenation in the presence of 10% palladium-on-carbon to obtain a compound of formula (I/b), a particular case of the compounds of formula (I), 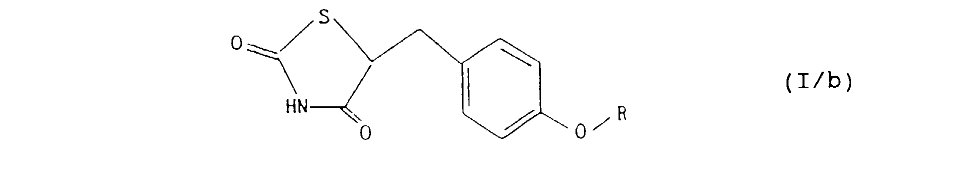 wherein R is as defined hereinbefore,
which compounds of formula (I/a) and (I/b) are purified, where appropriate, using a conventional purification method, and are separated into their isomers, if desired, using a conventional separating method, and are converted, if necessary, into their addition salts with a pharmaceutically acceptable acid.

2. Process for the preparation of the compounds of formula (I) according to claim 1 wherein R represents a substituted or unsubstituted [1-(benzothiazol-2-yl)pyrrolidin-2-yl]methyl group, the enantiomers, diastereoisomers and epimers thereof and also the addition salts thereof with a pharmaceutically acceptable acid.

3. Process for the preparation of the compounds of formula (I) according to claim 1 wherein R represents a substituted or unsubstituted [1-(benzoxazol-2-yl)pyrrolidin-2-yl]methyl group, the enantiomers, diastereoisomers and epimers thereof and also the addition salts thereof with a pharmaceutically acceptable acid.
